# EUROPEAN PATENT APPLICATION

(11) **EP 2 993 177 A1**
(43) Date of publication of application: **09.03.2016**
(21) Application number: 14183836.7
(22) Date of filing: 05.09.2014
(51) Int. Cl.: C07H 19/06, C07H 19/16, C07H 1/00, C12P 19/38, C12N 9/10

(54) **A chemo-enzymatic preparation method for purine nucleosides and their deaza- and aza- analogues**

(71) Applicant: Technische Universität Berlin, 10623 Berlin (DE)
(72) Inventor: Zhou, Xinrui, 10243 Berlin (DE)
(74) Representative: Gulde & Partner

(57) **Abstract**

The present invention relates to a new, chemo-enzymatic preparation method of purine nucleosides and their deaza- and aza analogues of general formula I, or pharmaceutically acceptable esters or salts thereof 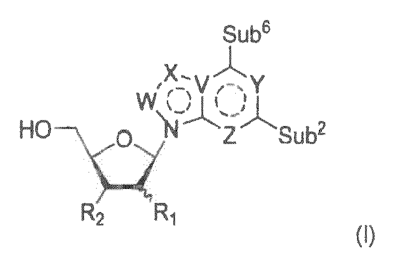 wherein
X, Y, Z and V independently from each other represent a nitrogen or carbon atom, W represents a nitrogen atom, -CH, -CF or -C-NH₂,
R₁ represents hydrogen, fluorine (ribo/arabino), -OH (ribo/arabino) or -NH₂ (ribo) and R₂ represents -OH, -NH₂ or hydrogen and
Sub⁶ represents hydrogen, chlorine, fluorine, -NH₂, -NHR, -N₃, -OH, -OR, -SH or -SR, wherein R is alkyl, aryl or acyl and
Sub² represents hydrogen, chlorine, fluorine, -N₃, -NH₂, -NHR, -OH, -OR,
wherein R is alkyl, aryl or acyl.

## Description

The present invention relates to a new, chemo-enzymatic preparation method of purine nucleosides and their deaza- and aza analogues of general formula I, or pharmaceutically acceptable esters or salts thereof 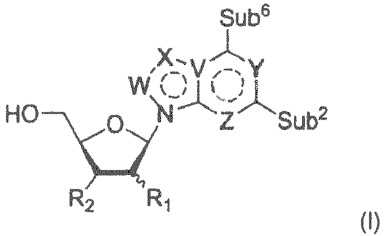 wherein
**X, Y, Z and V** independently from each other represent a nitrogen or carbon atom,
**W** represents a nitrogen atom, -CH, -CF or -C-NH₂,
**R₁** represents hydrogen, fluorine (ribo/arabino), -OH (ribo/arabino) or -NH₂ (ribo) and
**R₂** represents -OH, -NH₂ or hydrogen and
**Sub⁶** represents hydrogen, chlorine, fluorine, -NH₂, -NHR, -N₃, -OH, -OR, -SH or -SR,
   wherein R is alkyl, aryl or acyl and
**Sub²** represents hydrogen, chlorine, fluorine, -N₃, -NH₂, -NHR, -OH, -OR,
   wherein R is alkyl, aryl or acyl
which uses 2- or 6-halogenated or 2,6-dihalogenated nucleosides or their analogues of general formula II 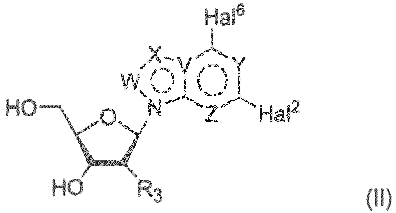 wherein
**X, Y, Z, V** and **W** have the above mentioned meaning,
**R₃** represents -OH or hydrogen,
**Hal², Hal⁶** represents hydrogen, fluorine, chlorine or bromine ,
as precursors. These starting compounds have good solubility in aqueous medium at already 25-40 °C. With these precursors the purine nucleosides and deaza- and aza analogues of formula I can be selectively obtained in a simple, efficient and cost-effective chemo-enzymatic route with 2 to 3 steps as described below.

Another object of the present invention is the provision of an advantageous preparation method for the starting 2,6-dihalogenated purine nucleosides and their deaza- and aza-analogues of general formula II which preparation method ensures high yields.

Purine nucleosides are of potential biological importance. Among the purine nucleosides of formula I are anti-tumour and anti-viral agents. Especially adenosine analogues containing halogen at the C2 carbon atom and an amino group at the C6 carbon atom are of potential interest. Indeed, Cladribine (2-chloro-2'-deoxyadenosine), Clofarabine (2-chloro-9-(2-deoxy-2-fluoro-ß-D-arabinofuranosyl) adenine) and Fludarabine (2-fluoro-9-(ß-D-arabinofuranosyl) adenine) attracted much attention during the last two decades as drugs of great therapeutic potential, in particular for the treatment of leukemia (T. Robak et al., Curr. Pharm. Des. 2012, 18, 3373-3388). 2-chloroadenosine was shown to be an agonist of the adenosine receptor that induces apoptosis in several cell lines. 2'-Deoxy-2-fluoroadenosine and 2-fluoroadenosine are the first members of this group of adenosine analogues which have been synthesized in the middle of the last century. They showed high cytostatic activity against tumour cells.

Until now, the vast majority of purine nucleosides and analogues is synthesized by chemical methods which result in yields less than 50% and/or comprise laborious operations to protect the functional groups of the sugar moiety and to isolate the obtained product from isomers. The production of Cladribine (2-chloro-2'-deoxyadenosine) as described in EP 173059 A2 and WO 2006/13396 A2 is one of these examples.

Two approaches to the synthesis of purine nucleosides have been widely and extensively studied. The first, convergent approach consists in the condensation of purine base with a suitable pentofuranose derivative and subsequent deprotection. The second, comprises the chemical transformation of purine base or/and pentofuranose fragment of the natural commercially available ribo- or 2'-deoxyribo- nucleoside in the desired compound.

That means, despite the very impressive progress achieved in the development of chemical methods, the preparation of purine nucleosides remains a challenging problem as of the indispensable introduction and subsequent removal of protective groups, which is associated with chromatographic purification on almost every step, a lack of selectivity of chemical reactions, and the problems associated with the poor regio- and stereo- selectivity of chemical reactions.

Considerable progress in the preparation of purine nucleosides like e.g. Cladribine was achieved by the combination of chemical methods and biochemical transformation. Thus, Vladimir N. Barai et al. describe in Helvetica Chimica Acta, Vol. 85 (2002), pages 1901-1908 the preparation of Cladribine via enzymatic transglycosylation using 2'-deoxyguanosine as sugar moiety donor and 2-chloroadenine as acceptor in the presence of glutaraldehyde-treated *E.coli* BMT 4D/1A cells as biocatalyst. These cells possess high UPase (uridine phosphorylase) and PNPase (purine nucleoside phophorylase) activities. They were crosslinked by glutaraldehyde to retain these high UPase and and PNPase activities. The drawback of this preparation method is the extremely low solubility of the 2-chloroadenine.

EP 1 932 918 A1 describes a production method of Cladribine which also uses the enzymatic transglycosylation in the presence of UPase and PNPase between 2-deoxyuridine as sugar moiety donor and 2-chloradenine as acceptor. This method also suffers from the extremely low solubility of the 2-chloroadenine as starting compound.

It was the object of the present invention to provide a simple, efficient and cost-effective preparation method for purine nucleosides and their deaza- and aza analogues which method avoids the use of the corresponding purine base as precursor and replace it by precursors which are easier to handle.

Accordingly, the present invention provides a chemo-enzymatic method for preparing purine nucleosides and their deaza- and aza-analogues of general formula I, or pharmaceutically acceptable esters or salts thereof 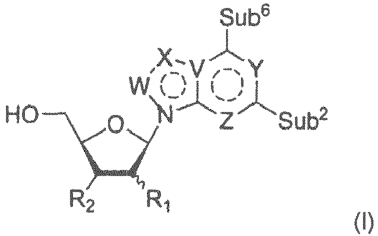 wherein
**X, Y, Z and V** independently from each other represent a nitrogen or carbon atom,
**W** represents a nitrogen atom, -CH, -CF or -C-NH₂,
**R₁** represents hydrogen, fluorine (ribo/arabino), -OH (ribo/arabino) or -NH₂ (ribo) and
**R₂** represents -OH, -NH₂ or hydrogen and
**Sub⁶** represents hydrogen, chlorine, fluorine, -NH₂, -NHR, -N₃, -OH, -OR, -SH or -SR,
   wherein R is alkyl, aryl or acyl and
**Sub²** represents hydrogen, chlorine, fluorine, -N₃, -NH₂, -NHR, -OH, -OR,
   wherein R is alkyl, aryl or acyl,
which is characterised in that
2,6-dihalogenated or 2- or 6- halogenated ribo- or 2'-deoxyribo nucleosides of general formula II 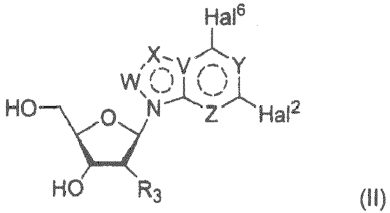 wherein
**X, Y, Z, V** and **W** have the above mentioned meaning,
**R₃** represents -OH or hydrogen,
**Hal², Hal⁶** represents hydrogen, fluorine, chlorine or bromine ,
are reacted in step 1 in a nucleophilic substitution reaction to ribo- or 2'-deoxyribo nucleosides of general formula III 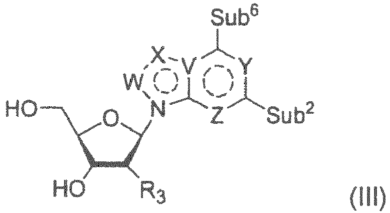 wherein
**X, Y, Z, V, W, Sub⁶**, **Sub²** and **R₃** have the above mentioned meaning,
or pharmaceutically acceptable esters or salts thereof,
and, optionally, further to modify the sugar moiety,
the nucleosides of general formula III are reacted in step 2 by enzymatic transglycosylation in the presence of biocatalysts with a nucleoside of general formula IV in the presence of phosphate buffer 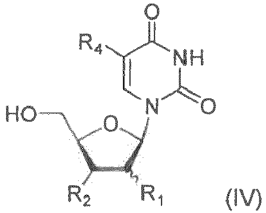 wherein
**R4** is hydrogen or methyl and
**R₁** and **R₂** have the above mentioned meaning
or
with pentofuranase-1-phosphate of formula V 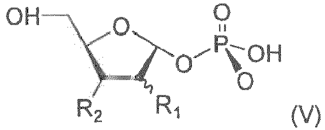 wherein
**R₁** and **R₂** have the above mentioned meaning
to purine nucleosides and their analogues of general formula I, or pharmaceutically acceptable esters or salts thereof, wherein step 1 and step 2 are exchangeable.

The change of the sequence of step 1 and step 2 means that the nucleosides of formula II may first be reacted by enzymatic transglycosylation with a nucleoside of general formula IV or with pentofuranose-1-phosphate of formula V to modify the sugar moiety and then they are reacted with a nucleophile to form the final purine nucleoside or its deaza-or aza-analogue of formula I

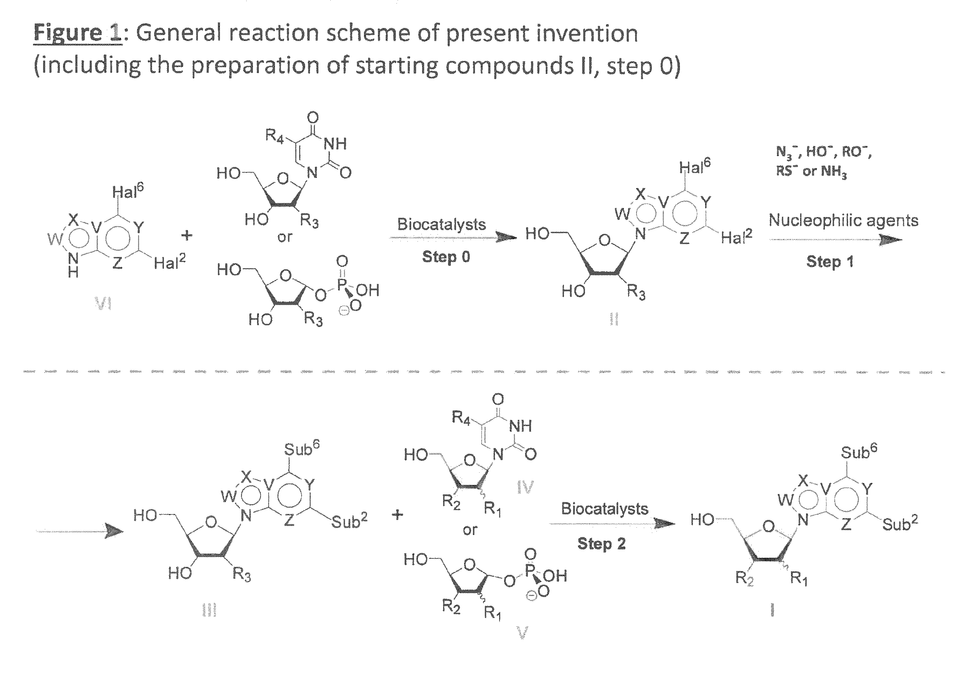

The general reaction scheme of the method of present invention is shown in Figure 1.

According to the present invention alkyl means a branched or unbranched C1-C4 chain, preferably methyl or ethyl. Aryl refers to an aromatic carbocyclic group having at least one aromatic ring (e.g. phenyl) or multiple condensed rings in which at least one ring is aromatic (e.g. naphthyl, thienyl or indolyl). An acyl group refers to any group or radical of the form RCO- where R is alkyl or aryl, e.g. acetyl chloride (CH₃COCl) and benzoyl chloride (C₆H₅COCl).

The preferred esters of the purine nucleosides of the invention are mono-, di- and triphosphate esters. The pharmaceutically acceptable salts of the compounds of formula I are preferably acid addition salts derived from an appropriate acid, e.g. hydrochloric, sulphuric, phosphoric, maleic, fumaric, citric, tartaric, lactic, acetic or p-toluenesulphonic acid. Particularly preferred salts are the hydrochloride salts of purine nucleosides and analogues of formula (I)

According to the present invention the soluble 2- or 6-halogenated or 2,6-dihalogenated, ribo- or 2'-deoxyribo purine nucleosides or analogues of general formula II are used as precursors for the preparation of the ribo- or 2'-deoxyribo nucleosides of formula III which are the base donors (key substrates) for the subsequent transglycosylation reaction instead of using as base donors the hardly soluble 2,6-dihalogenated or 2-halogenated bases directly.

According to one embodiment of the invention 2- or 6- halogenated or 2,6-dihalogenated ribo-or 2'-deoxyribo nucleosides of formula II with X, Y and Z being a nitrogen atom, V being a carbon atom and W being -CH, -CF or -C-NH₂, preferably -CH, are used as precursors to prepare the corresponding purine nucleosides of formula I.In formula II of the used 2,6-dihalogenated ribo- or 2'-deoxyribo nucleosides Hal⁶ is preferably chlorine and Hal² is chlorine or fluorine.

In an especially preferred embodiment of the invention 6-chloro-2-fluoropurine riboside (6C2FP-R), 6-chloro-2-fluoropurine-2'-deoxyriboside (6C2FP-dR), 2,6-dichloropurine riboside (26DCP-R) or 2,6-dichloropurine-2'-deoxyriboside (26DCP-dR) are used as precursors of formula II.

According to one embodiment of the present invention purine nucleosides or analogues of formula I with Sub⁶ being -NH₂ or -OH and Sub² being chlorine or fluorine are prepared. According to another preferred embodiment of the present invention purine nucleosides or analogues of formula I with Sub⁶ being chlorine or fluorine and Sub² being -NH₂ are prepared

According to other embodiments of the present invention deaza- and aza-analogues of purine nucleosides of formula I are prepared.

In some embodiments 8-azapurines of formula I (X, Y, Z and W representing a nitrogen atom and V representing a carbon atom), 8-aza-7-deazapurines of formula I (Y, Z and W being nitrogen and X and V being a carbon atom) and 5-aza-7-deazapurines of formula I (Y, Z and V being nitrogen and X and W representing a carbon atom) are prepared according to the described chemo-enzymatic method of the present invention.

According to a preferred embodiment of the present invention 6-halogenated 8-aza-7-deazapurine-ribosides of general formula II are used as precursors to produce the corresponding 6-substituted 8-aza-7-deazapurines of formula I.

In other embodiments of the invention 1-deazapurines (X and Z representing a nitrogen atom and Y, V and W being a carbon atom), 3-deazapurines (X and Y representing a nitrogen atom and Z, V and W being a carbon atom) and 1,3-dideazapurines (X being nitrogen and Y, Z, V and W being a carbon atom) are prepared according to the described chemo-enzymatic method.

According to the preparation method of the present invention the compounds of formula II are reacted in step 1 in a nucleophilic substitution reaction to compounds of formula III. The nucleophilic substitution at C6 and C2 atoms of the nucleosides of formula II can be carried out as well known in the art with an appropriate nucleophilic agent such as a negatively charged anion (e.g. N₃⁻, HO⁻, RO⁻, RS⁻) or a compound containing a free pair of electrons (e.g.NH₃) in an aprotic solvent such as e.g. dioxane or dimethoxyethane.

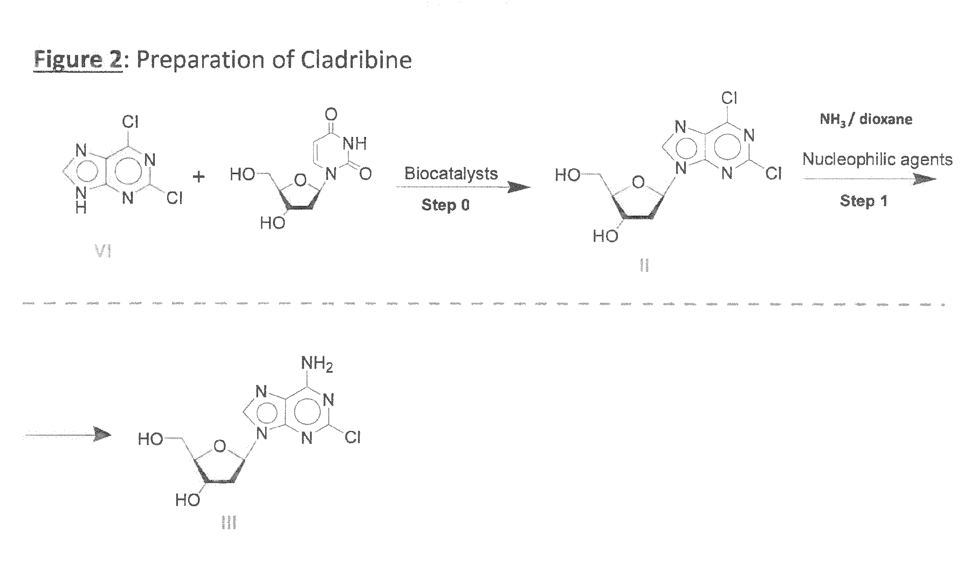

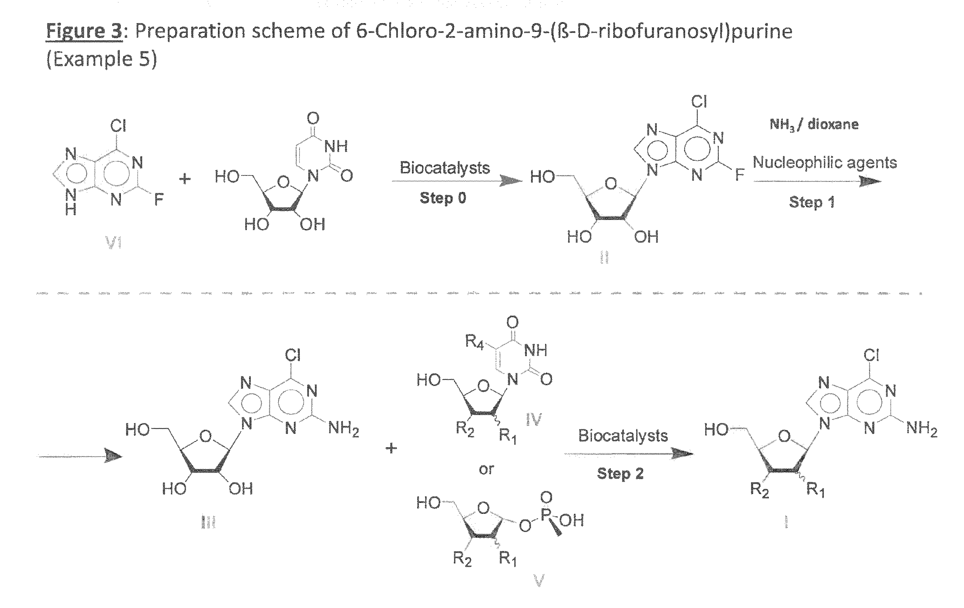

In one embodiment of step 1 of the present invention 2,6-dihalogenated nucleosides of general formula II are reacted by ammonolysis in one step to nucleosides of general formula III with Sub⁶ or Sub² being -NH₂ (compare Figures 2 and 3). The ammonolysis is preferably carried out by contacting the dihalogenated nucleosides of general formula II with ammonia in an organic solvent, preferably dioxane or dimethoxyethane, at ambient temperature until the reaction is completed, usually within 8 to 28 hours.In another embodiment of step 1 of the present invention nucleosides of general formula III with Sub⁶ or Sub² being -NH₂ which are obtained by ammonolysis as described above can be further reacted by oxidative deamination to nucleosides of general formula III with Sub⁶ or Sub² being -OH. Adenosine deaminase (EC.3.5.4.4) is preferably used for oxidative deamination.

In yet another embodiment of step 1 of the present invention nucleosides of general formula III with Sub⁶ or Sub² being Cl can be further reacted by dechlorination to nucleosides of general formula III with Sub⁶ or Sub² being -OH.

According to step 2 of the present invention the sugar moiety of the obtained ribo- or 2'-deoxyribo nucleosides of formula III can be modified in an enzymatic transglycosylation reaction with a respective pentofuranosyl donor in the presence of biocatalysts. In the enzymatic transglycosylation reaction of nucleosides of general formula III with nucleosides of general formula IV nucleoside phosphorylases (NPs) or nucleoside deoxyribosyltransferases (NDTs) can be used as biocatalysts. If R₁ in formula IV represents hydrogen NDTs can be used. But in all cases NPs are preferable over NDTs: (i) in the case of pyrimidine 2'-deoxyribonucleosides as pentofuranose donors there is great offer of enzymes, viz., either uridine, or thymidine, or pyrimidine phosphorylases can be used and(ii) the efficiency of these NPs is high and the substrate specificity is broad.

In a preferred embodiment of the present invention the biocatalysts used in the enzymatic transglycosylation reaction of step 2 are immobilized. The immobilisation can be done e.g. on magnetic microsphere beads carrying epoxide or amine groups or the enzymes are immobilised by other means well known in the art, e.g. over fusion tags (e.g. HIS-tag) or by chemical cross-linking. In one embodiment of the present invention the biocatalysts are immobilised on magnetic microsphere beads. In another embodiment of the present invention the biocatalysts which are used in step 2 are GtPNP (purine nucleoside phosphorylase from *Geobacillus thermoglucosidans*) and GtPyNP (pyrimidine nucleoside phosphorylase from *Geobacillus thermoglucosidans*) or GtPNP and TtPyNP (pyrimidine nucleoside phosphorylase from *Thermus thermophilus*) which are co-immobilized on magnetic microsphere beads.

Another object of the present invention is the preparation of the 2- or 6- halogenated or 2,6-dihalogenated ribo- or 2'-deoxyribo purine nucleosides and their deaza- and aza-analogues of formula II in high yields which are precursors for the preparation of the purine nucleosides and their analogues of formula I.

The 2- or 6- halogenated or 2,6-dihalogenated ribo- or 2'-deoxyribo purine nucleosides and their deaza- and aza- analogues of general formula II 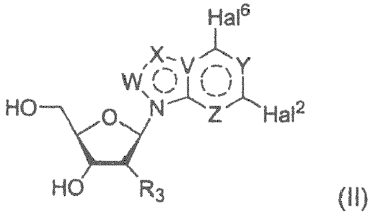 wherein
**X, Y, Z and V** independently from each other represent a nitrogen or carbon atom,
**W** represents a nitrogen atom, -CH, -CF or -C-NH₂,
**R₃** represents -OH or hydrogen and
**Hal²,** Hal⁶ represents hydrogen, fluorine, chlorine or bromine
are prepared by reacting 2- or 6- halogenated or 2,6-dihalogenated purines or purine analogues of general formula VI 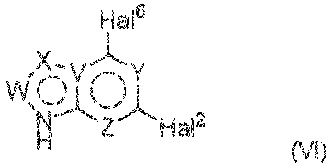 wherein **X, Y, Z, V, W, Hal⁶** and **Hal²** have the above mentioned meaning
in a transglycosylation reaction in the presence of biocatalysts with
a) a nucleoside of general formula VII in the presence of phosphate buffer 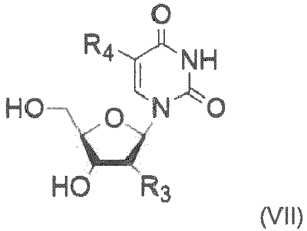 wherein
   **R₃** has the above mentioned meaning and
   **R₄** represents hydrogen or methyl,
   or
b) pentofuranose-1-phosphate of general formula VIII 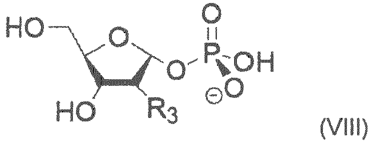 wherein
   **R₃** has the above mentioned meaning,
to 2- or 6-halogenated or 2,6-dihalogenated ribo- or 2'-deoxyribo purine nucleosides and their analogues of general formula II, wherein the transglycosylation reaction is performed at 45 to 80°C with immobilized purine nucleoside phosphorylase (PNP) and pyrimidine nucleoside phosphorylase (PyNP) as biocatalysts.

The used purine nucleoside phosphorylase is preferably from *Geobacillus thermoglucosidans* (GtPNP) and the used pyrimidine nucleoside phosphorylase is preferably from *Thermus thermophilus* (TtPyNP). Both are thermostable enzymes and accept halogenated substrates. In a preferred embodiment of the present transglycosylation reaction the enzymes are immobilized on magnetic microsphere beads, preferably beads carrying epoxide groups. In an especially preferred embodiment the enzymes are immobilized on MagReSynß™ Epoxide Microspheres (ReSyn Biosciences, South Africa). In a particularly preferred embodiment of present reaction the used GtPNP and TtPyNP are co-immobilized.

Using magnetic microsphere beads carrying epoxide groups high immobilization yield with high enzyme loading of the particularly used enzymes could be obtained. TtPyNP and GtPNP were loaded up to 0.38 and 1.32 g per g dry beads, respectively, with high enzyme activity maintenance (41% and 83%, respectively).

For the synthesis of compounds of formula II, the substrates pyrimidine nucleoside (uridine or thymidine) of formula VII and the halogenated purine bases of formula VI are preferably reacted in the molar ratio of about 2:1 to 3:1. The concentration of phophate buffer is preferably 1-5mM.

It revealed that under the described reaction conditions of high substrate concentration and using the selected and particularly immobilized enzymes the product yields of 2,6-Dichloropurine riboside (26DCP-R) and 6-Chloro-2-fluoropurine riboside (6C2FP-R) were increased from 54% and 63% to 78% and 85%, respectively.

According to the invention it is preferred to use 2,6-dihalogenated purines or purine analogues of general formula VI with X, Y and Z being a nitrogen atom, V being a carbon atom and W being -CH, -CF or -C-NH₂, preferably W being -CH, as starting compounds.

In formula VI of the used 2,6-dihalogenated purines or purine analogues Hal⁶ is preferably chlorine and Hal² is fluorine or chlorine. 6-chloro-2-fluoropurine (6C2FP) and 2,6-dichloropurine (26DCP) are the especially preferred starting compounds of formula VI for the preparation of the corresponding dihalogenated ribosides and 2'-deoxyribosides of formula II.

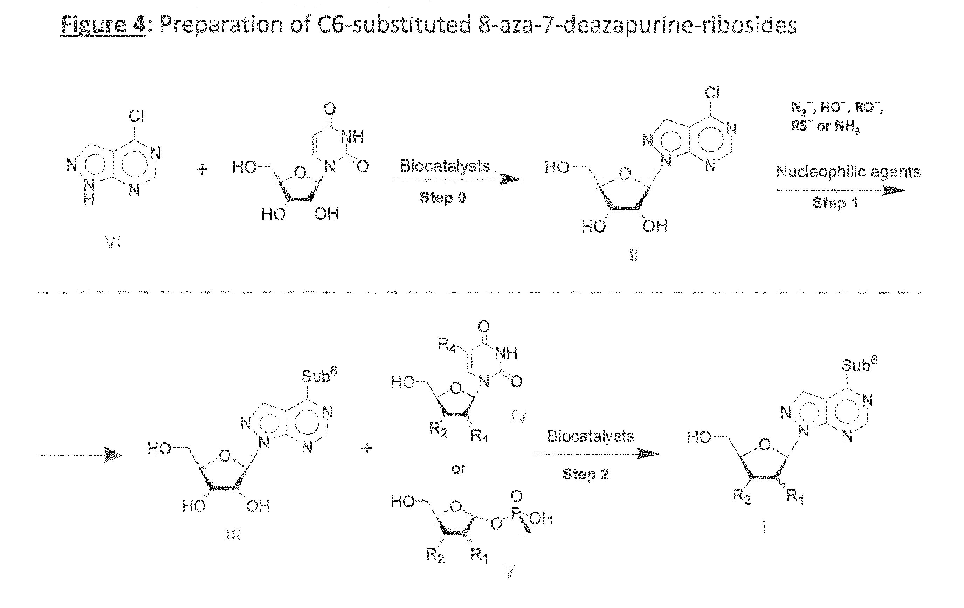

According to another preferred embodiment of the present invention 6-halogenated purines or purine analogues of general formula VI with X, Y and Z being a nitrogen atom, V being a carbon atom and W being -CH, -CF or -C-NH₂, preferably W being -CH, are used as starting compounds. It is also preferred to use 6-halogenated 8-aza-7-deazapurine-ribosides of general formula II as precursors to produce the corresponding 6-substituted 8-aza-7-deazapurines of formula I (compare Figure 4).
**Figure 1** shows the general reaction scheme for the preparation of purine nucleosides and their deaza- and aza- analogues according to present invention.
**Figure 2** shows the reaction scheme for the preparation of Cladribine according to present invention.
**Figure 3** shows the reaction scheme for the preparation of 6-Chloro-2-aminopurine riboside according to present invention.
**Figure 4** shows the reaction scheme for the preparation of C6-substituted 8-aza-7-deazapurine-ribosides according to present invention.

The following examples are offered to illustrate the described preparation methods. The examples are not intended to be limiting in any respect.

### Example 1:

### Enzyme immobilisation

TtPyNP (or GtPyNP) and GtPNP were immobilized on MagEM separately according to the manual (http://www.resynbio.com) with a slight modification. Briefly, 20 µL suspension beads (25 mg mL⁻¹) were washed and equilibrated in binding buffer (50 mM K-phosphate buffer, pH 6) shortly before using. Enzyme solution (PyNP and GtPNP can be loaded up to 0.4 and 1.3 g per g dry beads, respectively) with the binding buffer was adjusted to 10 times volume of the initial beads suspension, i.e. 200 µL of total volume. The enzyme and beads suspension were mixed thoroughly and were put on the thermomixer at 50 °C and shaking at 950 rpm for 4 h. After binding procedure, the beads were collected by magnetic separator and the supernatant was discarded or used for quantification of immobilization yield. The beads were washed with washing buffer (50 mM K-phosphate buffer, pH 7.0; 1 M NaCl) for three times to remove non-covalently bound enzyme. Residual epoxide groups were quenched using a similar procedure, except that a quenching solution (1 M Tris pH 9.0) instead of the binding solution was used with incubation for 1.5 h. The beads were washed with 50 mM K-phosphate buffer pH 7.0 for three times and were ready to use or were stored at 4 °C.

Co-immobilisation of TtPyNP and GtPNP was performed under these conditions in the same way of the individual enzyme immobilisation, but the coupling enzymes were first admixed and then added into the beads in the binding step.

### Example 2:

### Preparation of 6-Chloro-2-fluoro-9-(β-D-ribofuranosyl)purine (6C2FP-R)

Reaction mixture (50 mL) containing uridine 50 mM (0.61 g, 2.5 mmol), 6-chloro-2-fluoropurine 25 mM (0.216 g, 1.25 mmol), potassium phosphate 2 mM (0.1 mmol, pH 7.0) and immobilized biocatalysts (TtPyNP: 8 mg; GtPNP: 15 mg) was enclosed in a 50 mL-falcon tube and rotated by an end-over-end rotator at 55-60 °C in a water bath for 20 h. 75 % conversion on the base was determined by HPLC. The biocatalyst was removed by magnet and the reaction mixture was concentrated under vacuum. The residues were purified on a silica gel column chromatography (dichloromethane: ethanol= 15:1). However, 6C2FP-R and uracil were not separated successfully and the related fractions were concentrated and subjected to a second silica gel purification (ethyl acetate: acetone= 7:3). Finally the desired product was purified and obtained as a white solid (0.230 g, 0.75 mmol; HPLC purity 98 %) in 60% yield.

Melting point= 182-183 °C (1^{st} run), 182-183.5 °C (2^{nd} run)

Chemical shifts of ¹H and ¹³C NMR are reported in parts per million (ppm) downfield from tetramethylsilane and are referenced to the residual solvent resonance as the internal standard (DMSO: δ = 2.54 ppm for ¹H and DMSO-*d*₆: δ = 40.45 ppm for ¹³C). Data are reported as follows: chemical shift, multiplicity (br s= broad singlet, s= singlet, d= doublet, t= triplet, dt= doublet of triplets, q= quartet, m= multiplet), coupling constants (Hz) and integration.

**¹H NMR** (500 MHz, DMSO-*d*₆):
δ = 8.99 (s, 1 H; H-8), 5.98 (d, 1H, *J*_{1',2'} = 4.9 Hz; H-1'), 5.63 (d, *J =* 5.7 Hz, 1H; 2'-OH), 5.29 (d, *J =* 5.5 Hz, 1H; 3'-OH), 5.11 (t, *J* = 5.5 Hz, 1 H; 5'-OH), 4.54 (m, 1 H, H-2'), 4.22 (m, 1 H; H-3'), 4.03 (dt, 1 H, *J*_{4',3'}= 4.03 Hz, *J*_{4',5'}= *J*_{4',5'} = 4.01 Hz; H-4'), 3.74 (ddd, ^{gem}*J* = 12.0 Hz, *J* = 5.2 Hz, *J* = 4.1 Hz, 1 H), 3.63 (ddd, ^{gem}*J* = 11.9 Hz, *J* = 5.1 Hz, *J* = 4.0 Hz, 1 H) ppm.

**¹³C NMR** (126 MHz, DMSO-*d*₆):
δ = 157.08 (d *J* = 213.96 Hz; C-2), 154.48 (d, *J* = 19.5 Hz; C-6(4)), 151.49 (d, *J =* 18.2 Hz; C-4(6)), 147.27 (d, *J =* 2.51 Hz; C-8 through 5 bonds), 131.4 (d, *J =* 4.8 Hz; C-5), 89.2 (C-1), 86.6 (C-4) 74.9 (C-2), 70.8 (C-3), 61.7 (C-5) ppm.

**HR**-**MS**(APCI) exact mass *m*/*z*= 305.0450, calcd. for C₁₀H₁₀ClFN₄O₄ [M+H]⁺: 305.0447. Isotope: calcd. for [M+H]⁺*m*/*z*=307.0418, found: 307.0420.

### Example 3:

### Preparation of 2,6-Dichloropurine-2'-deoxyriboside (26DCP-dR)

Reaction mixture (0.4 mL) containing uridine 2 mM (0.195 mg, 0.8 µmol), 2,6-dichloropurine 1 mM (0.076 mg, 0.4 µmol), potassium phosphate 2 mM (0.8 µmol, pH 7.0) and non-immobolized enzymes GtPyNP and GtPNP (0.1 mg mL⁻¹ each). Reaction was performed in a 1.5 mL eppi tube on a thermomixer at 65 °C for 0.5 h (the negative control without enzyme was performed in parallel and showed no product formation). 67 % conversion on the base was determined by HPLC. New peak was observed by HPLC and the product confirmation was done by LC-MS.

### Verification of 26DCP-dR via LC-MS

Reaction mixture was analyzed with Agilent 6460 TripleQuad LC-MS mass spectrometer in combination with Agilent 1290 Infinity HPLC system equipped with the Zorbax Eclipse Plus C18 RRHD column (1.8 µm, 50 x 2.1 mm) using ACN and H₂O + 0.1% HCOOH as mobile phase. The compounds were analyzed during a linear gradient starting at 5% ACN till 50% ACN over 9 min and a re-equilibration phase at 5% ACN for 1 min with a flow rate of 0.3 mL min⁻¹. MS conditions were set to ESI positive ion mode. The mass range was 100-400 m/z.

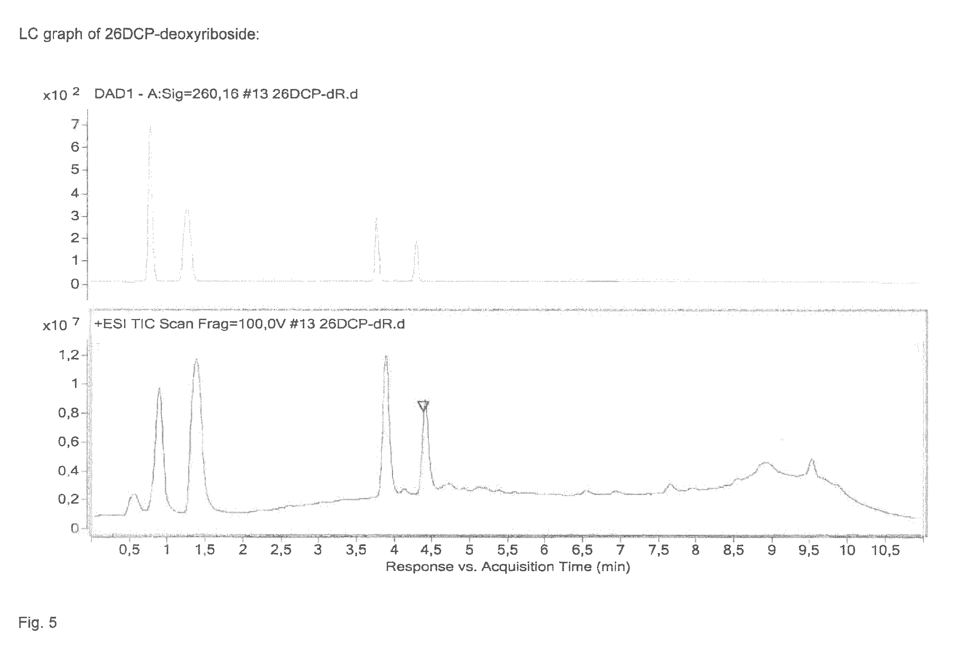

The LC graph of 26DCP-deoxyriboside is shown in Figure 5.

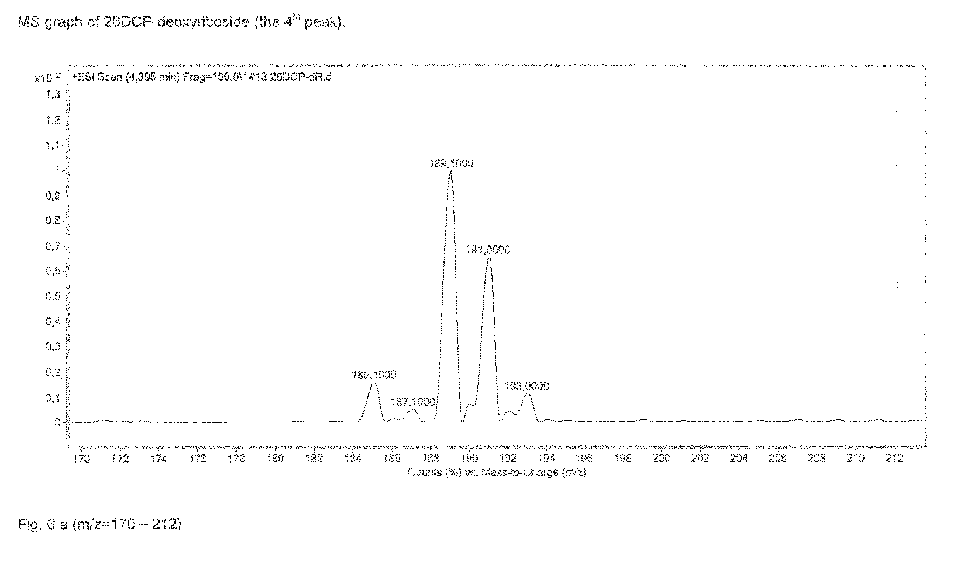

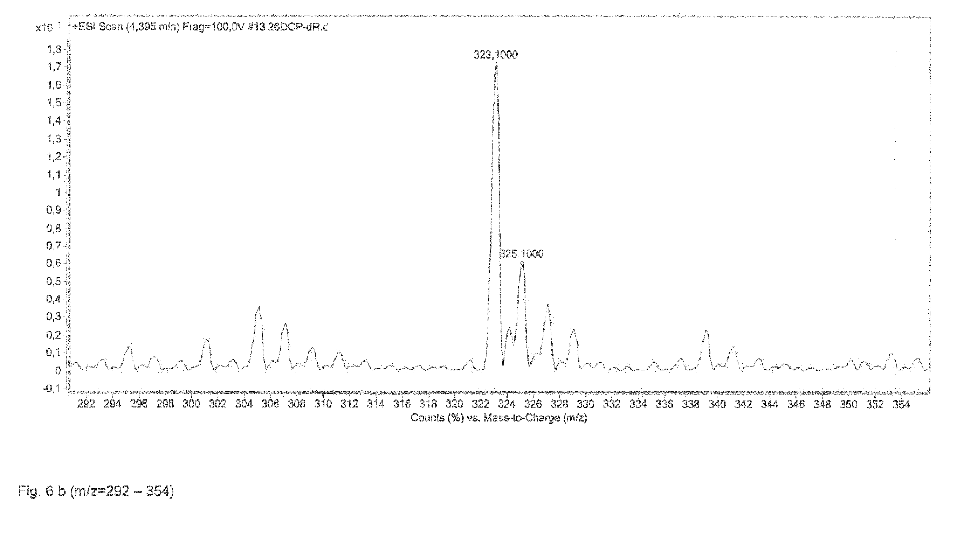

The MS graph of 26DCP-deoxyriboside (the 4^{th} peak) is shown in Figure 6.

### Example 4:

### Preparation of 6-Chloro-8-aza-7-deazapurine-riboside

Reaction mixture (0.2 mL) containing 6-chloro-8-aza-7-deazapurine 1 mM (0.031 mg, 0.2 µmol), ribosie-1-phosphate bis(cyclohexylammonium) salt 1 mM (0.086 mg, 0.2 µmol), and the immobolized enzyme GtPNP (*ca*. 0.1 mg mL⁻¹). Reaction was performed in a 1.5 mL eppi tube on a thermomixer at 65 °C, 1000 rpm for 6 h (the negative control without enzyme was performed in parallel and showed no product formation). 62 % conversion on the base was determined by HPLC. New peak was observed by HPLC and the product confirmation was done by LC-MS.

### Verification of 6-chloro-8-aza-7-deazapurine-riboside via LC-MS

Full-scan measurements were performed on an Exactive ESI-Orbitrap-MS (Thermo Fisher Scientific GmbH, Bremen, Germany) coupled to an analytical Agilent HPLC 1200 system (Agilent, Waldbronn, Germany) equipped with Chromsil120 ODS-4HI column (5 µm, 50 x 2.1 mm, Grace, Columbia, USA) using ACN and H₂O + 0.1% HCOOH as mobile phase. The compounds were analyzed during a linear gradient starting at 5% ACN till 50% ACN over 10 min followed by a washout phase 100% ACN for 4 min and a re-equilibration phase at 5% ACN for 2 min The flow rate was set to 0.3 mL min⁻¹. MS conditions were set to two micro scans with R = 10,000 (200 m/z) with a maximal C trap fill time of 100 ms.

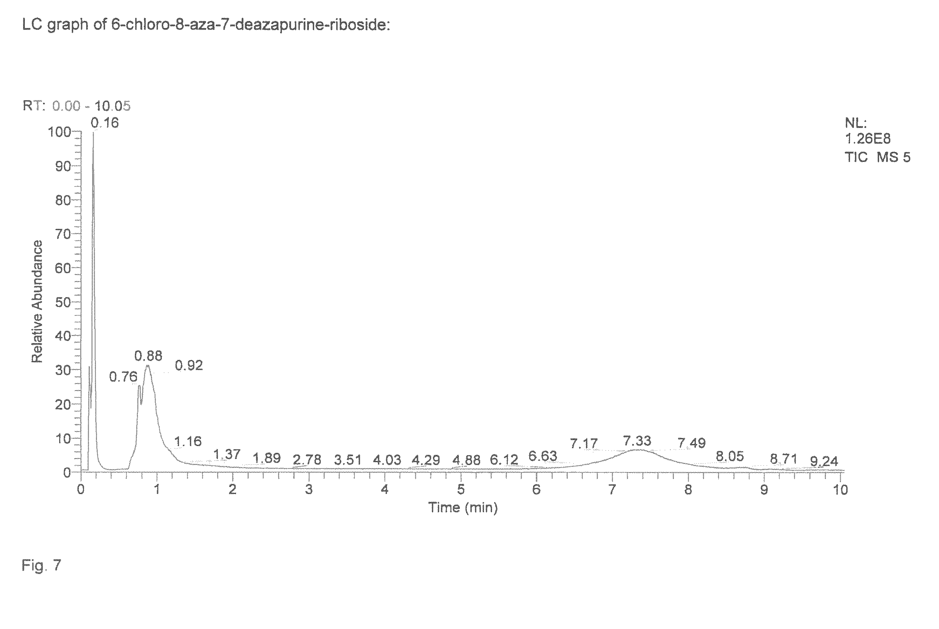

The LC graph of 6-chloro-8-aza-7-deazapurine-riboside is shown in Figure 7.

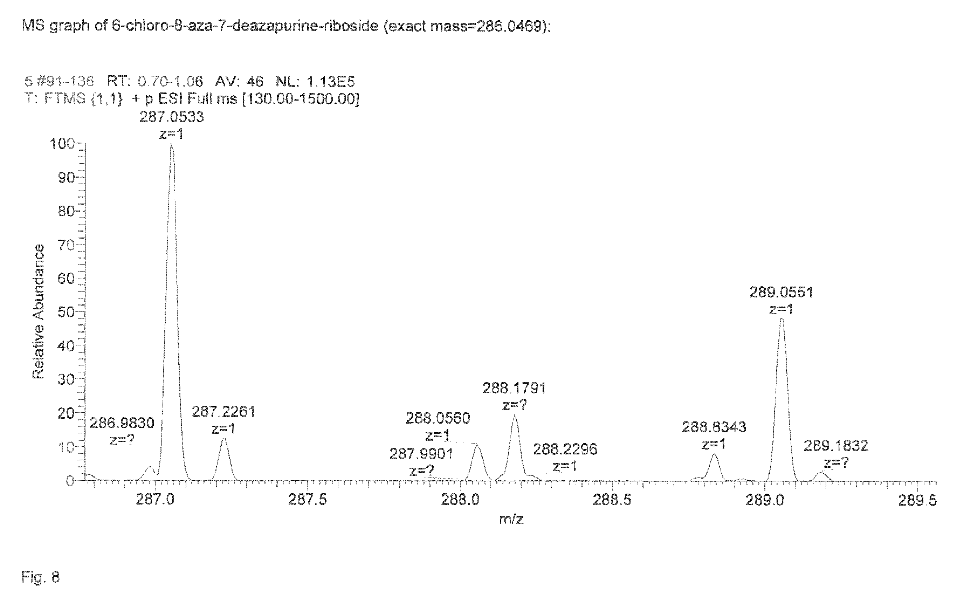

The MS graph of 6-chloro-8-aza-7-deazapurine-riboside (exact mass=286.0469) is shown in Figure 8

### Example 5:

### Preparation of 6-Chloro-2-amino-9-(ß-D-ribofuranosyl)purine by ammonolysis of 6C2FP-R

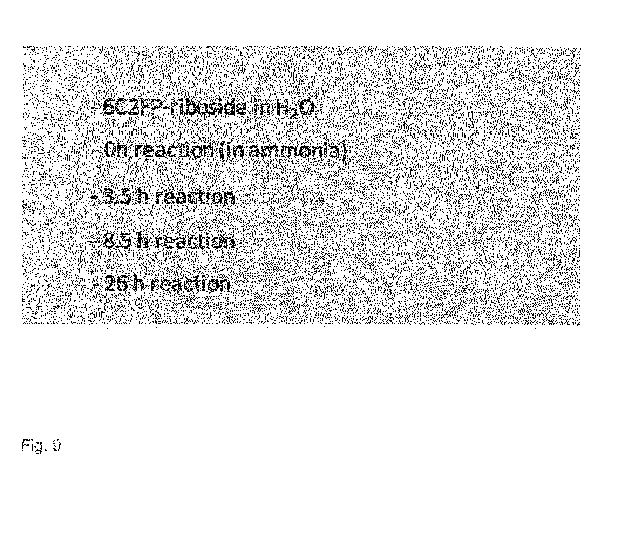

6C2FP-R prepared in Example 2 (8.1 mg) was dissolved in 1 mL 0.5 M ammonia solution in dioxane (Sigma-aldrich) in glass tube with a rubber stopper. Reaction was performed at room temperature for 26 h and the reaction process was monitored by TLC with tetrahydrofuran + 0.5 % NH₃•H₂O as eluent. TLC results are shown in Figure 9.

### Verification of 6-Chloro-2-amino-9-(ß-D-ribofuranosyl)purine via LC-MS

Full-scan measurements were performed on an Exactive ESI-Orbitrap-MS (Thermo Fisher Scientific GmbH, Bremen, Germany) coupled to an analytical Agilent HPLC 1200 system (Agilent, Waldbronn, Germany) equipped with Chromsil120 ODS-4HI column (5 µm, 50 x 2.1 mm, Grace, Columbia, USA) using ACN and H₂O + 0.1% HCOOH as mobile phase. The compounds were analyzed during a linear gradient starting at 5% ACN till 50% ACN over 10 min followed by a washout phase 100% ACN for 4 min and a re-equilibration phase at 5% ACN for 2 min. The flow rate was set to 0.3 mL min⁻¹. MS conditions were set to two micro scans with R = 10,000 (200 m/z) with a maximal C trap fill time of 100 ms.

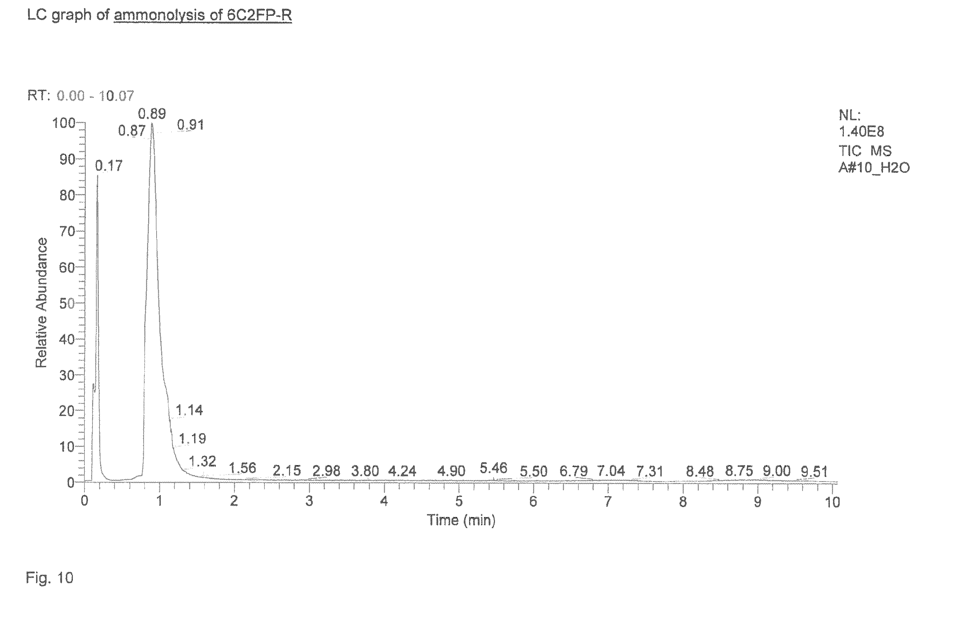

The LC graph of ammonolysis of 6C2FP-R is shown in Figure 10.

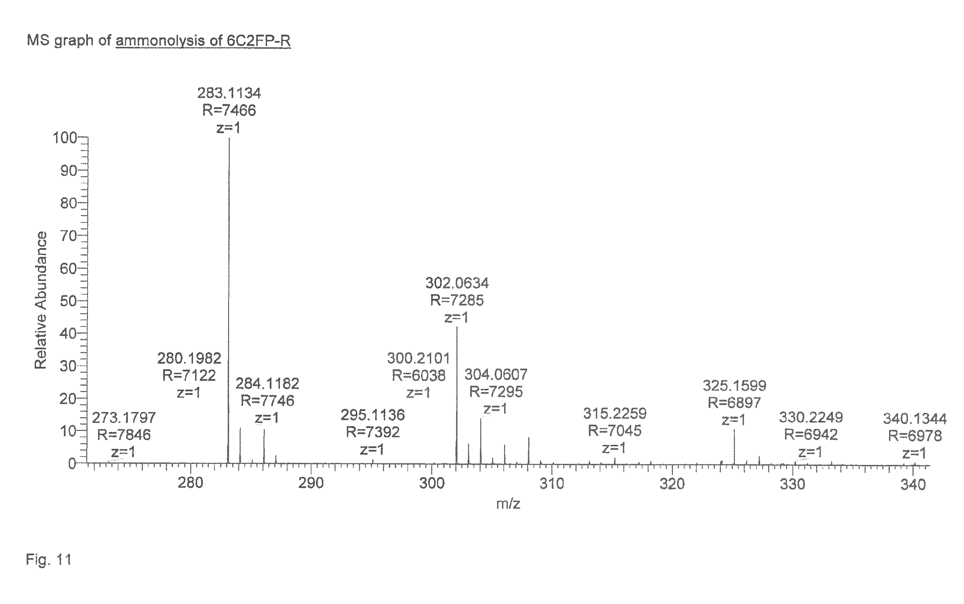

The MS graph of ammonolysis of 6C2FP-R is shown in Figure 11.

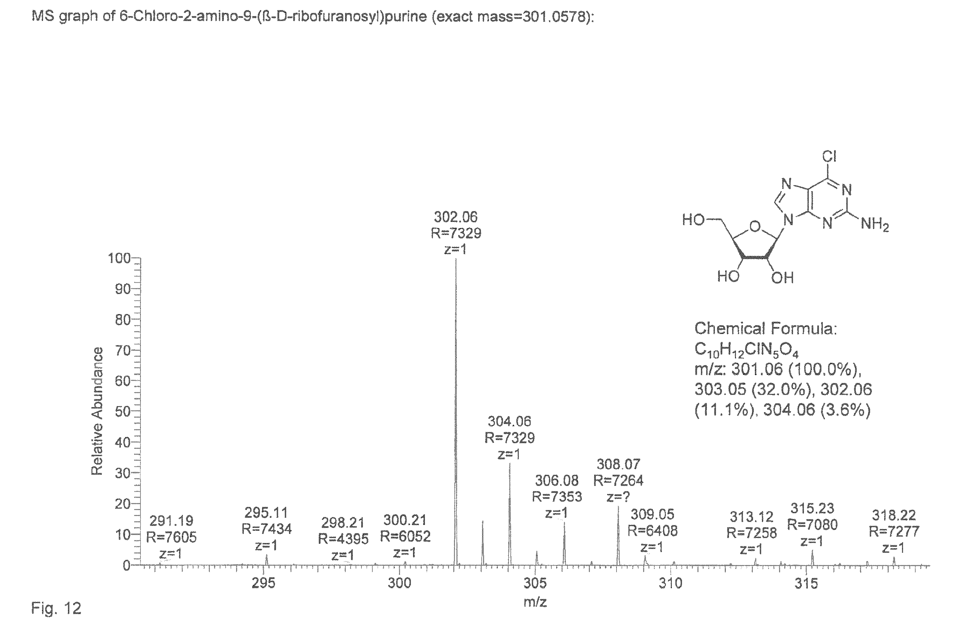

The MS graph of 6-Chloro-2-amino-9-(ß-D-ribofuranosyl)purine (exact mass=301.0578) is shown in Figure 12.

## Claims

1. A chemo-enzymatic method for preparing purine nucleosides and their deaza- and aza-analogues of general formula I, or pharmaceutically acceptable esters or salts thereof 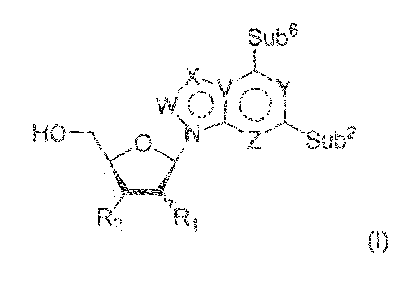 wherein
**X**, **Y, Z and V** independently from each other represent a nitrogen or carbon atom,
**W** represents a nitrogen atom, -CH, -CF or -C-NH₂,
**R₁** represents hydrogen, fluorine (ribo/arabino), -OH (ribo/arabino) or -NH₂ (ribo) and
**R₂** represents -OH, -NH₂ or hydrogen and
**Sub⁶** represents hydrogen, chlorine, fluorine, -NH₂, -NHR, -N₃, -OH, -OR, -SH or -SR,
wherein R is alkyl, aryl or acyl and
**Sub²** represents hydrogen, chlorine, fluorine, -N₃, -NH₂, -NHR, -OH, -OR,
wherein R is alkyl, aryl or acyl,
**characterised in that**
2,6-dihalogenated or 2- or 6-halogenated ribo- or 2'-deoxyribo nucleosides of general formula II 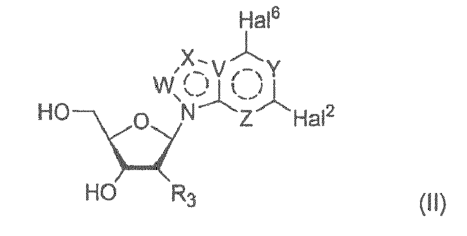 wherein
**X, Y, Z, V** and **W** have the above mentioned meaning,
**R₃** represents -OH or hydrogen,
**Hal⁶, Hal²** represent hydrogen, fluorine, chlorine or bromine are reacted in step 1 in a nucleophilic substitution reaction to ribo- or 2'-deoxyribo nucleosides of general formula III 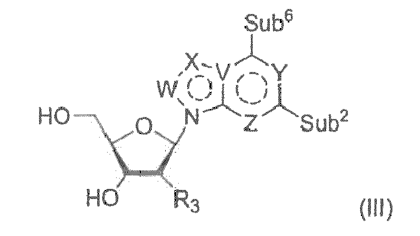
wherein
**X, Y, Z, V, W**, **Sub⁶, Sub²** and **R₃** have the above mentioned meaning,
or pharmaceutically acceptable esters or salts thereof,
and, optionally,
the nucleosides of general formula III are reacted in step 2 in an enzymatic transglycosylation reaction in the presence of biocatalysts and phosphate buffer with a nucleoside of general formula IV 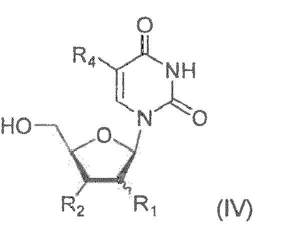 wherein
**R4** is hydrogen or methyl and
**R₁** and **R₂** have the above mentioned meaning
or
with pentofuranose-1-phosphate of formula V 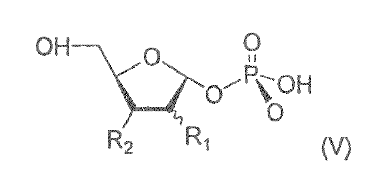 wherein
**R₁** and **R₂** have the above mentioned meaning
to purine nucleosides and their analogues of general formula I, or pharmaceutically acceptable esters or salts thereof, wherein step 1 and step 2 are exchangeable

2. . The method according to claim 1,
**characterised in that**
purine nucleosides and their analogues of general formula I with Sub⁶ being -NH₂ or -OH and Sub² being chlorine or fluorine or with Sub⁶ being chlorine or fluorine and Sub² being -NH₂ are prepared.

3. The method according to claim 1 or 2,
**characterised in that**
purine nucleosides of general formula I with X, Y and Z being a nitrogen atom, V being a carbon atom and W being -CH, -CF or -C-NH₂ are prepared.

4. . The method according to any one of claims 1 to 3,
**characterised in that**
the 2,6-dihalogenated or 2- or 6-halogenated ribo- and 2'-deoxyribo nucleosides of general formula II are reacted in step 1 by ammonolysis to ribo- and 2'-deoxyribo nucleosides of general formula ill with Sub⁶ being -NH₂ and Sub² being chlorine or fluorine.

5. . The method according to any one of claims 1 to 4,
**characterised in that**
nucleosides of general formula III with Sub⁶ being -NH₂ and Sub² being chlorine or fluorine are reacted in step 1 by oxidative deamination to nucleosides of general formula III with Sub⁶ being -OH and Sub² being chlorine or fluorine.

6. . The method according to claim 5,
**characterised in that**
adenosine deaminase (ADA) is used for oxidative deamination of nucleosides of general formula III with Sub⁶ being -NH₂ and Sub² being chlorine or fluorine.

7. . The method according to any one of claims 1 to 6,
**characterised in that**
in the enzymatic transglycosylation reaction of nucleosides of general formula III with nucleosides of general formula IV in step 2 nucleoside phosphorylases (NPs) or nucleoside deoxyribosyltransferases (NDTs) are used as biocatalysts.

8. . The method according to any one of claims 1 to 7,
**characterised in that**
in the enzymatic transglycosylation reaction of nucleosides of general formula III with pentofuranose-1-phosphate of formula V in step 2 nucleoside phosphorylases (NPs) are used as biocatalysts

9. The method according to any one of claims 1 to 8,
**characterized in that**
the biocatalysts used in the enzymatic transglycosylation reaction of step 2 are immobilized, preferably on magnetic microsphere beads carrying epoxide or amine groups.

10. . A method for preparing 2- or 6-halogenated or 2,6-dihalogenated ribo- or 2'-deoxyribo
purine nucleosides and their deaza- and aza- analogues of general formula 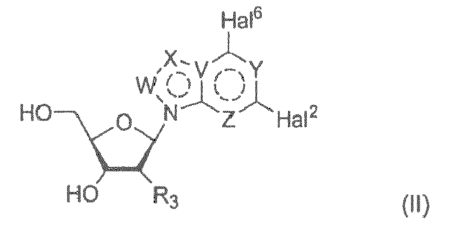 wherein
**X, Y, Z and V** independently from each other represent a nitrogen or carbon atom, **W** represents a nitrogen atom, -CH, -CF or -C-NH₂,
**R₃** represents -OH or hydrogen,
**Hal⁶, Hal²** represent hydrogen, fluorine, chlorine or bromine
by reacting 2- or 6-halogenated or 2,6-dihalogenated purines or purine analogues of general formula VI 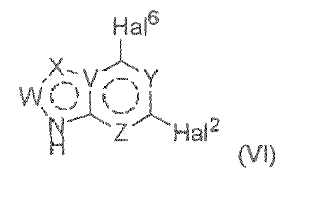 wherein **X, Y, Z, V, W, Hal⁶** and **Hal²** have the above mentioned meaning
in a transglycosylation reaction in the presence of biocatalysts with
a) a nucleoside of general formula VII in the presence of phosphate buffer 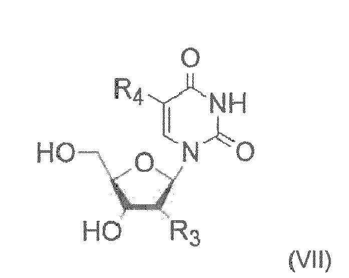 wherein
**R₃** has the above mentioned meaning and
**R₄** represents hydrogen or methyl,
or
b) pentofuranose-1-phosphate of general formula VIII 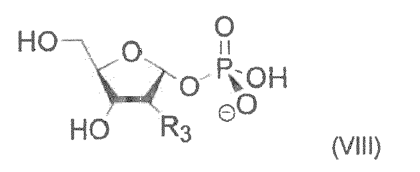 wherein
**R₃** has the above mentioned meaning,
to 2- or 6-halogenated or 2,6-dihalogenated ribo- or 2'-deoxyribo purine nucleosides and their analogues of general formula II,
**characterised in that**
the transglycosylation reaction is performed at 45-80°C with immobilized purine nucleoside phosphorylase (PNP) and pyrimidine nucleoside phosphorylase (PyNP) as biocatalysts.

11. . The method according to claim 10,
**characterised in that**
the used purine nucleoside phosphorylase is from *Geobacillus thermoglucosidans* (GtPNP) and the used pyrimidine nucleoside phosphorylase is from *Thermus thermophilus* (TtPyNP) or *Geobacillus thermoglucosidans (GtPyNP).*

12. . The method according to claims 10 or 11,
**characterised in that**
the used purine nucleoside phosphorylase and pyrimidine nucleoside phosphorylase are immobilized on magnetic microsphere beads carrying epoxide groups, preferably GtPNP and TtPyNP are co-immobilized on these beads.

13. . The method according to any one of claims 10 to 12,
**characterized in that**
2,6-dihalogenated purines or purine analogues of general formula VI with X, Y and Z being a nitrogen atom, V being a carbon atom and W being -CH, -CF or -C-NH₂, preferably W being -CH, are used as starting compounds.

14. . The method according to any one of claims 10 to 13,
**characterized in that**
in the used 2,6-dihalogenated purines or purine analogues of formula VI Hal⁶ is chlorine and Hal² is fluorine or chlorine.

15. . Use of 2,6-dihalogenated or 2- or 6-halogenated ribo- or 2'- deoxyribo nucleosides of general formula II 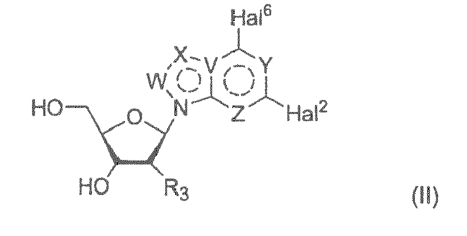 wherein
**X, Y, Z and V** independently from each other represent a nitrogen or carbon atom,
**W** represents a nitrogen atom, -CH, -CF or -C-NH₂,
**R₃** represents -OH or hydrogen,
**Hal⁶, Hal²** represent hydrogen, chlorine or bromine
as precursors for preparing purine nucleosides and their deaza- and aza-analogues of general formula I, or pharmaceutically acceptable esters or salts thereof 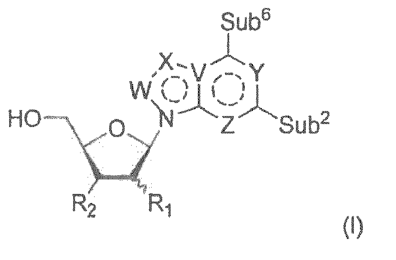 wherein
**X, Y, Z, V** and **W** have the mentioned meaning,
**R₁** represents hydrogen, fluorine (ribo/arabino), -OH (ribo/arabino) or -NH₂ (ribo) and
**R₂** represents -OH, -NH₂ or hydrogen and
**Sub⁶** represents hydrogen, chlorine, fluorine, -NH₂, -NHR, -N₃, -OH, -OR, -SH or -SR,
wherein R is alkyl, aryl or acyl and
**Sub²** represents hydrogen, chlorine, fluorine, -N₃, -NH₂, -NHR, -OH, -OR,
wherein R is alkyl, aryl or acyl.
